(19) Europäisches Patentamt European Patent Office Office européen des brevets

(11) **EP 4 362 330 B1**

(12) **EUROPEAN PATENT SPECIFICATION**

(45) Date of publication and mention
of the grant of the patent:
**29.10.2025  Bulletin 2025/44**

(21) Application number: **21966630.2**

(22) Date of filing: **06.12.2021**

(51) International Patent Classification (IPC):
*A61B 5/30* (2021.01)     *H03F 1/14* (2006.01)
*H03F 3/45* (2006.01)     *H03F 1/56* (2006.01)

(52) Cooperative Patent Classification (CPC):
**H03F 3/45475; A61B 5/30; H03F 1/14; H03F 1/56;**
H03F 2200/222; H03F 2200/261; H03F 2203/45138;
H03F 2203/45151; H03F 2203/45156;
H03F 2203/45512; H03F 2203/45528;
H03F 2203/45546; H03F 2203/45548;
H03F 2203/45594; H03F 2203/45601;       (Cont.)

(86) International application number:
**PCT/CN2021/135877**

(87) International publication number:
**WO 2023/102702 (15.06.2023 Gazette 2023/24)**

(54) **SIGNAL ACQUISITION CIRCUIT AND WEARABLE DEVICE**

SIGNALERFASSUNGSSCHALTUNG UND WEARABLE-VORRICHTUNG

CIRCUIT D'ACQUISITION DE SIGNAL ET DISPOSITIF PORTABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**01.05.2024  Bulletin 2024/18**

(73) Proprietor: **Shenzhen Shokz Co., Ltd.
Shenzhen, Guangdong 518108 (CN)**

(72) Inventors:
• **DENG, Wenjun
  Shenzhen, Guangdong 518108 (CN)**
• **ZHANG, Yuxiang
  Shenzhen, Guangdong 518108 (CN)**
• **LIAO, Fengyun
  Shenzhen, Guangdong 518108 (CN)**
• **QI, Xin
  Shenzhen, Guangdong 518108 (CN)**

(74) Representative: **Wang, Bo
  Panovision IP
  Ebersberger Straße 3
  85570 Markt Schwaben (DE)**

(56) References cited:
  CN-A- 102 697 500     CN-A- 103 928 842
  CN-A- 109 981 060     CN-A- 111 464 150
  CN-A- 113 495 589     CN-U- 204 854 720

• **SAAD MOHAMED ET AL: "A chopper capacitive
  feedback instrumentation amplifier with input
  impedance boosting technique", 2016 IEEE 59TH
  INTERNATIONAL MIDWEST SYMPOSIUM ON
  CIRCUITS AND SYSTEMS (MWSCAS), IEEE, 16
  October 2016 (2016-10-16), pages 1 - 4,
  XP033073109, DOI: 10.1109/
  MWSCAS.2016.7870153**
• **ZHANG SANFENG ET AL: "23.7 A 130dB CMRR
  Instrumentation Amplifier with Common-Mode
  Replication", 2020 IEEE INTERNATIONAL SOLID-
  STATE CIRCUITS CONFERENCE - (ISSCC), IEEE,
  16 February 2020 (2020-02-16), pages 356 - 358,
  XP033754321, DOI: 10.1109/
  ISSCC19947.2020.9062928**

EP 4 362 330 B1

- **ABDELFATTAH SAFAA ET AL: "A Chopper Instrumentation Amplifier with Fully Symmetric Negative Capacitance Generation Feedback Loop and Online Digital Calibration for Input Impedance Boosting", 2019 IEEE 62ND INTERNATIONAL MIDWEST SYMPOSIUM ON CIRCUITS AND SYSTEMS (MWSCAS), IEEE, 4 August 2019 (2019-08-04), pages 774 - 777, XP033652920, DOI: 10.1109/ MWSCAS.2019.8884858**
- **LEE JINSEOK ET AL: "An Ultra-High Input Impedance Analog Front End Using Self-Calibrated Positive Feedback", vol. 53, no. 8, 1 August 2018 (2018-08-01), USA, pages 2252 - 2262, XP093197885, ISSN: 0018-9200, Retrieved from the Internet <URL:https://ieeexplore.ieee. org/stampPDF/getPDF.jsp?tp=& arnumber=8364561& ref=aHR0cHM6Ly9pZWVleHBsb3JlLmllZ WUub3JnL2RvY3VtZW50LzgzNjQ1NjE=> [retrieved on 20240822], DOI: 10.1109/ JSSC.2018.2831231**

- **SISINNI EMILIANO ET AL: "Full-Analog Parasitic Capacitance Compensation for AC-Excited Differential Sensors", IEEE TRANSACTIONS ON INSTRUMENTATION AND MEASUREMENT, IEEE, USA, vol. 69, no. 8, 1 January 2020 (2020-01-01), pages 5890 - 5899, XP011794879, ISSN: 0018-9456, [retrieved on 20200623], DOI: 10.1109/ TIM.2019.2962296**

(52) Cooperative Patent Classification (CPC): (Cont.)
H03F 2203/45604; H03F 2203/45632;
H03F 2203/45698

## Description

### TECHNICAL FIELD

The present disclosure relates to the field of circuit design, and in particular to a signal acquisition circuit and a wearable device.

### BACKGROUND

In existing bioelectric signal acquisition circuits, such as signal acquisition circuits for electrocardiography (ECG) and electromyography (EMG), a common-mode power frequency signal is often converted into a differential-mode power frequency noise input amplifier, leading to a reduction in the signal-to-noise ratio and even circuit saturation failure. Consequently, a higher input impedance is required to reduce power frequency interference. However, in practical circuits, a parasitic capacitance often causes a decrease in the input impedance of the circuit, thereby exacerbating power frequency interference. In other words, the presence of the parasitic capacitance limits the effectiveness of bioelectric signal acquisition.

Therefore, there is a need to provide a signal acquisition circuit and a wearable device that can minimize the impact of the parasitic capacitance on signal acquisition. CN 109 981 060 A relates to a method for improving noise and input impedance of capacitance-coupled chopper instrumentation amplifier. SAAD MOHAMED ET AL: "A chopper capacitive feedback instrumentation amplifier with input impedance boosting technique", 2016 IEEE 59TH INTERNATIONAL MIDWEST SYMPOSIUM ON CIRCUITS AND SYSTEMS (MWSCAS), IEEE, 16 October 2016, pages 1-4, XP033073109, DOI: 10.1109/MWSCAS.2016.7870153 [retrieved on 2017-03-02] relates to a chopper capacitive feedback instrumentation amplifier (CCFIA) with proposed input impedance boosting technique. ZHANG SANFENG ET AL: "23.7 A 130dB CMRR Instrumentation Amplifier with Common-Mode Replication", 2020 IEEE INTERNATIONAL SOLID-STATE CIRCUITS CONFERENCE - (ISSCC), IEEE, 16 February 2020, pages 356-358, XP033754321, DOI: 10.1109/ISSCC19947.2020.9062928 [retrieved on 2020-04-09] relates to a 130dB CMRR instrumentation amplifier with common-mode replication. ABDELFATTAH SAFAA ET AL: "A Chopper Instrumentation Amplifier with Fully Symmetric Negative Capacitance Generation Feedback Loop and Online Digital Calibration for Input impedance Boosting", 2019 IEEE 62ND INTERNATIONAL MIDWEST SYMPOSIUM ON CIRCUITS AND SYSTEMS (MWSCAS), IEEE, 4 August 2019, pages 774-777, XP033652920, DOI: 10.1109/MWSCAS.2019.8884858 [retrieved on 2019-10-28] relates to a symmetric chopper instrumentation amplifier architecture with two identical 8-bit digitally programmable capacitor banks and online digital calibration block.

### SUMMARY

The invention is set out in the appended set of claims.

### BRIEF DESCRIPTION OF THE DRAWINGS

The present disclosure is further illustrated by way of exemplary embodiments, which is described in detail through the accompanying drawings. These embodiments are not limiting, and in these embodiments, the same numbering indicates the same structure, wherein:

FIG. 1 is a schematic diagram illustrating the principle of power frequency interference, according to some embodiments of the present disclosure;
FIG.s 2A and 2B are schematic diagrams illustrating power supply modes of a differential amplifier, according to some embodiments of the present disclosure;
FIG. 3 is a schematic diagram illustrating a signal acquisition circuit, according to some embodiments of the present disclosure;
FIG. 4 is a structural schematic diagram of a negative capacitance circuit in a signal acquisition circuit, according to some embodiments of the present disclosure;
FIG. 5 is a schematic diagram illustrating a signal acquisition circuit, according to some embodiments of the present disclosure;
FIG. 6A is a structural schematic diagram of a fourth negative capacitance circuit in a signal acquisition circuit, according to some embodiments of the present disclosure;
FIG. 6B is another structural schematic diagram of the fourth negative capacitance circuit in the signal acquisition circuit, according to some embodiments of the present disclosure;
FIG. 7A is a schematic diagram illustrating an effect circuit before eliminating power frequency interference, according to some embodiments of the present disclosure; and

FIG. 7B is a schematic diagram illustrating an effect circuit after eliminating power frequency interference, according to some embodiments of the present disclosure.

## DETAILED DESCRIPTION

In order to more clearly illustrate the technical solutions of the embodiments of the present disclosure, the accompanying drawings for the description of the embodiments are described below. Obviously, the accompanying drawings in the following description are only some examples or embodiments of the present disclosure, and it is possible for a person of ordinary skill in the art to apply the present disclosure to other similar scenarios in accordance with these accompanying drawings without creative labor. Unless obviously obtained from the context or the context illustrates otherwise, the same numeral in the drawings refers to the same structure or operation.

It should be understood that the terms "system," "device," "unit," and/or "module" are used herein as a way to distinguish between different components, elements, parts, sections, or assemblies at different levels. However, if other words may achieve the same purpose, the terms may be replaced with alternative expressions.

As indicated in the present disclosure and in the claims, unless the context clearly suggests an exception, the words "one," "a," "a kind of," and/or "the" do not refer specifically to the singular but may also include the plural. In general, the terms "include" and "comprise" suggest only the inclusion of clearly identified steps and elements, which do not constitute an exclusive list, and the method or device may also include other steps or elements.

The signal acquisition circuit described in the present disclosure embodiment may be applied to various signal monitoring devices requiring signal acquisition, particularly monitoring devices for physiological signals, such as a smart wearable device. In some embodiments, the wearable device (e.g., a piece of clothing, a wristband, a shoulder strap, etc.) may be positioned on various parts of a human body (e.g., a calf, a thigh, the waist, the back, the chest, a shoulder, the neck, etc.) to collect physiological signals from different parts of a user's body in various states. Subsequently, the collected signals may be further processed. In some embodiments, the physiological signals are detectable signals that reflect the user's body status, including but not limited to respiratory signals, electrocardiographic (ECG) signals, electromyographic (EMG) signals, electroencephalographic (EEG) signals, blood pressure signals, temperature signals, etc. In some embodiments, the wearable device for collecting the physiological signals may be applied in emerging industries such as medical, gaming, entertainment, health education, etc. For example, the wearable device may be integrated with technologies like virtual reality (VR) and EMG collection to promote the development of immersive entertainment and education, or combined with technologies like mechatronics and exoskeletons to reduce medical costs and promote the development of medical health.

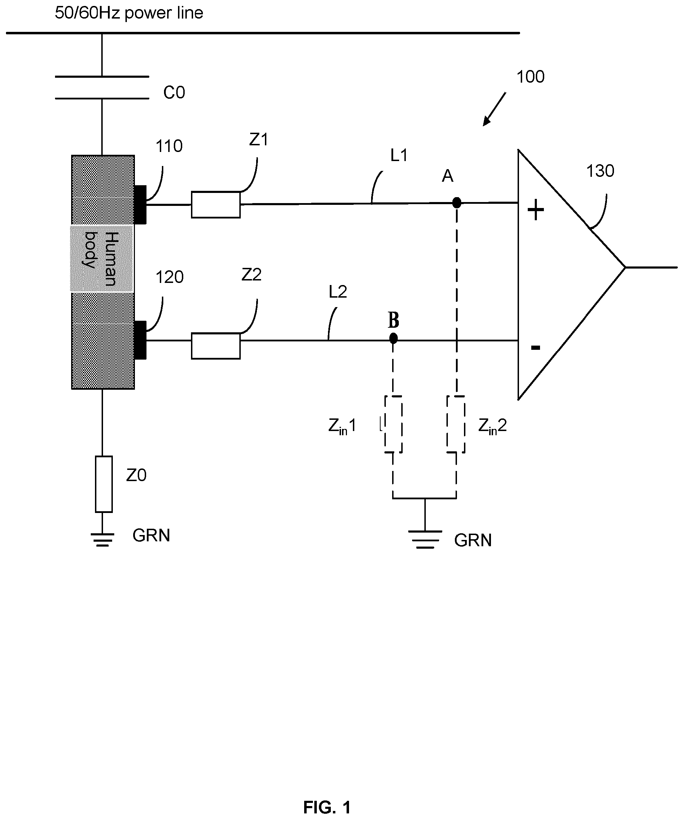

FIG. 1 is a schematic diagram illustrating the principle of power frequency interference, according to some embodiments of the present disclosure.

In some embodiments, as shown in FIG. 1, a signal acquisition circuit 100 includes one or more sensor units (e.g., a first electrode 110 and a second electrode 120) in contact with a user's body, a differential amplifier 130, and one or more leads (e.g., a first lead L1, a second lead L2) connecting the one or more sensor units to the differential amplifier 130.

In some embodiments, the one or more sensor units may be used to acquire one or more physiological signals from the user. The one or more sensor units may include, but are not limited to, an electromyographic sensor, a posture sensor, an electrocardiographic sensor, a respiratory sensor, a temperature sensor, a humidity sensor, an inertial sensor, a blood oxygen saturation sensor, a Hall sensor, a skin conductance sensor, a rotational sensor, etc., or any combinations thereof. In some embodiments, the physiological signals may include one or more of an electromyographic signal, a posture signal, an electrocardiographic signal, a respiratory rate, a temperature signal, a humidity signal, etc., or any combinations thereof. The one or more sensor units may be placed at different locations on the wearable device based on a type of motion signal to be acquired.

In some embodiments, different signal acquisition circuits may be arranged at different locations on the user's body to collect the same or different physiological signals. For example, signal acquisition circuits placed on different sides of the user's thighs may be used to collect electromyographic signals from the thighs. As another example, a signal acquisition circuit placed on the user's forearm may be used to collect an electromyographic signal from the forearm, while a signal acquisition circuit positioned at the user's heart may be used to collect an electrocardiographic signal.

In some embodiments, the sensor unit may include one or more electrode components in contact with the user's body, allowing the collection of an electromyographic signal from the surface of the user's body. The electrode component may be a dry electrode or a wet electrode. The dry electrode is a metallic structural electrode made of a metal sheet or a metal wire, while the wet electrode is formed by applying conductive gel between the human skin and the electrode component, enhancing the contact intensity with the human body. Since the human body is not a perfect conductor, both the dry and wet electrodes exhibit contact impedance with the human body. The impedance may vary under different physiological conditions, and different electrode types may be selected for different situations.

In some embodiments, the one or more electrode components may include the first electrode 110 and the second electrode 120. The first electrode 110 is connected to a first input terminal A of the differential amplifier 130 via the first lead L1, and the second electrode 120 is connected to a second input terminal B of the differential amplifier 130 via the second lead L2. The first lead L1 and the second lead L2 may transmit physiological signals collected by the one or more electrode components to the differential amplifier for appropriate processing (e.g., noise reduction, amplification, etc.).

In some embodiments, the differential amplifier 130 may differentially amplify the physiological signals obtained by the one or more electrode components. In some embodiments, the physiological signals processed by the differential amplifier 130, i.e., the $V_{out}$ outputted at an output terminal, may be passed to other components in the wearable device for further processing. For example, the processed physiological signals may be converted from analog to digital signals through an analog-to-digital converter (ADC) and then undergo further processing by a processor, such as signal analysis.

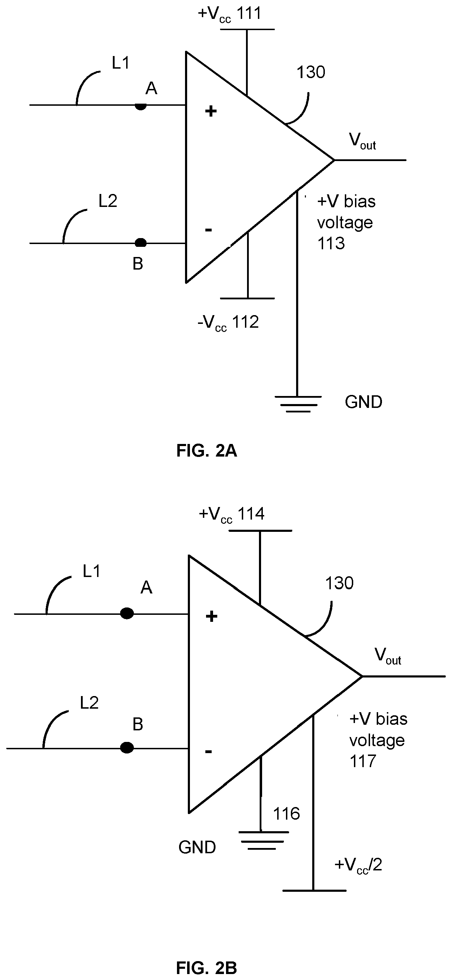

In some embodiments, the differential amplifier 130 may have two power supply modes: single-ended power supply and differential power supply. As shown in FIG. 2A, a first power supply terminal (111) of the differential amplifier 130 is connected to a positive power supply $+V_{cc}$, a second power supply terminal (112) is connected to a negative power supply $-V_{cc}$, and one end receiving a bias voltage (113) is grounded. As shown in FIG. 2B, a first power supply terminal (114) of the differential amplifier 130 is connected to the positive power supply $+V_{cc}$, a second power supply terminal (116) is grounded, and one end receiving the bias voltage (117) receives a bias voltage value of $+V_{cc}/2$.

In some embodiments, when the contact impedance between the first electrode 110 and the second electrode 120 and the user's body is inconsistent, i.e., when impedances at the two input terminals of the differential amplifier 130 are not the same (impedance imbalance), a common-mode power frequency signal on the body surface may be converted into a differential-mode power frequency noise inputted to the amplifier. This may reduce the signal-to-noise ratio, and in severe cases, it may even lead to circuit saturation and failure.

Due to the presence of various 50/60Hz power frequency power lines in the environment (50Hz in China and 60Hz abroad), the power frequency power lines emit electromagnetic waves that couple with the human body, causing changes in the body's potential. The amplitude of the changes is generally in the millivolt range, ranging from a few mV to several hundred mV, and is dependent on a position of the human body relative to the power lines. For ease of understanding, environmental interference may be modeled as a capacitive coupling model C0, and the impedance between the human body and the ground is represented as Z0. The model for power frequency interference may be equivalent to the left half of FIG. 1, where the power frequency power lines are connected in series with the equivalent capacitor C0, the human body, and the ground impedance. When the human body moves, the distance from the human body to the power lines changes, and the value of the equivalent capacitor C0 also changes.

In some embodiments, a power frequency current coupled to the human body may be denoted as $I_{cm}$, and a common-mode power frequency potential $V_{cm}$ on the surface of the human body may be expressed as:

$$V_{cm} = I_{cm} * Z0 \quad (1)$$

An input impedance to ground at the first input terminal A may be denoted as $Z_{in}1$, and an input impedance to ground at the second input terminal B may be denoted as $Z_{in}2$. Typically, $Z_{in}1 = Z_{in}2 = Z_{in}$. A common-mode input impedance may be expressed as by the following formula:

$$Z_{cm} = Z_{in}/2 \quad (2)$$

In some embodiments, when contact impedances of the first electrode 110 and the second electrode 120 with the user's body are not the same, it may lead to unequal potentials at the first input terminal A and the second input terminal B of the differential amplifier 130. The differential amplifier 130 may further amplify a potential difference between points A and B, leading to circuit saturation or failure. When there is a common-mode power frequency potential $V_{cm}$ on the human body, the potential difference $V_{AB}$ between the first input terminal A and the second input terminal B of the differential amplifier 130 may be represented by the following formula (3):

$$V_{AB} = V_A - V_B = V_{cm} * \frac{Z1-Z2}{Z_{in}} \quad (3)$$

wherein Z1 denotes the contact impedance value of the first electrode 110, and Z2 denotes the contact impedance value of the second electrode 120. From the above formula, it may be observed that a strength of the differential power frequency signal between the first input terminal A and the second input terminal B of the differential amplifier 130 is inversely proportional to the common-mode input impedance of the differential amplifier 130. The lower the common-mode input impedance, the greater the power frequency interference.

In some embodiments, to reduce the impact of power frequency interference on signal acquisition, techniques such as reducing the contact impedance of the electrode components may be employed to decrease the potential difference between the two input terminals of the differential amplifier. Additionally, a ground electrode to the human body may be added to decrease the common-mode power frequency potential, thereby reducing the potential difference between the two input terminals of the differential amplifier. For example, one end of the electrode may be in contact with the human body, while the other end is connected to the circuit's GND. However, as the electrode components always have contact impedance, it is impossible to completely eliminate the common-mode power frequency potential.

In some embodiments, to minimize the impact of power frequency interference on signal acquisition, a technique of increasing the (common-mode) input impedance of the differential amplifier may be employed. For example, a differential amplifier with high (common-mode) input impedance (e.g., both the first input terminal A and the second input terminal B having high input impedance to ground) may be used as an input front end of the signal acquisition circuit. In some embodiments, the (common-mode) input impedance of the differential amplifier 130 may be greater than 100M$\Omega$, and preferably, the (common-mode) input impedance of the differential amplifier 130 may be greater than 1G$\Omega$.

However, when the signal acquisition circuit 100 is applied to the wearable device, the presence of parasitic capacitance in an actual circuit may lead to a reduction in the input impedance of the differential amplifier. In practical applications, parasitic capacitance exists not only between leads but also between the leads and the ground. Especially in scenarios involving the collection of weak physiological signals, such as an electromyographic signals and an electrocardiographic signals, the impact of the parasitic capacitances becomes particularly pronounced. In some embodiments, the parasitic capacitances are connected in parallel with various equivalent impedances in the signal acquisition circuit, significantly reducing the overall input impedance of the circuit, thereby exacerbating power frequency interference. Furthermore, this issue may not be resolved by using a differential amplifier with higher input impedance. Thus, the presence of the parasitic capacitances limits the performance and effectiveness of the signal acquisition circuit.

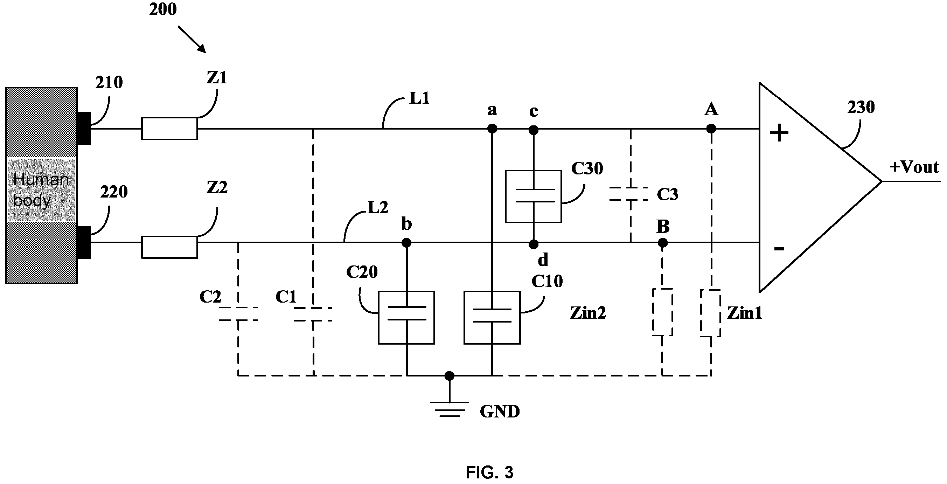

FIG. 3 is a schematic diagram of a signal acquisition circuit 200 according to some embodiments of the present disclosure.

In some embodiments, as shown in FIG. 3, the signal acquisition circuit 200 may include a first electrode 210, a second electrode 220, a differential amplifier 230, a first lead L1, a second lead L2, a first negative capacitor circuit C10, and a second negative capacitor circuit C20.

In some embodiments, the first electrode 210 may be connected to a first input terminal A of the differential amplifier 230 via the first lead L1, and the second electrode 220 may be connected to a second input terminal B of the differential amplifier 230 via the second lead L2. Relevant descriptions regarding the first electrode 210, the second electrode 220, the differential amplifier 230, the first lead L1, and the second lead L2 may be referred to in the corresponding descriptions in FIG. 1 and are not repeated here.

In some embodiments, there may be a first parasitic capacitance C1 to ground between the first lead L1 and the ground, a second parasitic capacitance C2 to ground between the second lead L2 and the ground, and a lead-to-lead parasitic capacitance C3 between the first lead L1 and the second lead L2. As shown in FIG. 3, the first parasitic capacitance C1 to ground may be considered equivalent to being connected between the first lead L1 and the ground, and the first parasitic capacitance C1 to ground is in parallel with a ground impedance $Z_{in}1$ of the first input terminal A of the differential amplifier 230. Similarly, the second parasitic capacitance C2 to ground may be considered equivalent to being connected between the second lead L2 and the ground, and the second parasitic capacitance C2 to ground is in parallel with a ground impedance $Z_{in}2$ of the second input terminal B of the differential amplifier 230. The lead-to-lead parasitic capacitance C3 may be considered equivalent to being connected between the first lead L1 and the second lead L2. Due to the parallel connection of the first parasitic capacitance C1 to ground and the second parasitic capacitance C2 to ground with the ground impedances $Z_{in}1$ and $Z_{in}2$ of the differential amplifier respectively, an overall input impedance of the circuit is greatly reduced, exacerbating the impact of power frequency interference on signal acquisition.

In some embodiments, the real-time measurement of a parasitic capacitance value is not feasible. However, since the parasitic capacitance value is related to circuit design, the parasitic capacitance value may be measured after a circuit is designed. In other words, values of the first parasitic capacitance C1 to ground, the second parasitic capacitance C2 to ground, and the lead-to-lead parasitic capacitance C3 may be measured after the circuit design of the signal acquisition circuit. In practical applications, it is challenging to precisely measure the parasitic capacitance values between the human body and the circuit. However, measurements may be performed when the human body is stationary to determine a baseline value of the parasitic capacitance. When the human body moves, causing a change in the parasitic capacitance value, an approximate range of fluctuations may be determined based on the baseline value.

In some embodiments, to mitigate the impact of the parasitic capacitance and increase the input impedance of the differential amplifier, the signal acquisition circuit 200 may include the first negative capacitor circuit C10 and the second negative capacitor circuit C20. The first negative capacitor circuit C10 is electrically connected between the first lead L1 and the ground, and the second negative capacitor circuit C20 is electrically connected between the second lead L2 and the ground. Both the first and second negative capacitor circuits C10 and C20 exhibit a negative capacitance effect, countering the lead-to-ground parasitic capacitance and reducing the impact of the parasitic capacitance on the circuit's input impedance and signal acquisition. This further enhances the performance and effectiveness of the signal acquisition circuit. The negative capacitance effect mentioned here may be understood as the trend of changes in the quantity of charge in the first negative capacitance circuit C10 and the second negative capacitance circuit C20 being opposite to the trend of changes in the voltage applied thereto. In other words, as the voltage decreases, the quantity of charge in both the first negative capacitance circuit C10 and the second negative capacitance circuit C20 increases accordingly.

In some embodiments, the manner of electrically connecting the first negative capacitor circuit C10 and/or the second negative capacitor circuit C20 to the ground may include connecting the first negative capacitor circuit C10 and/or the second negative capacitor circuit C20 to a common ground terminal on one or more printed circuit boards (PCBs).

In some embodiments, the first negative capacitor circuit C10 may be considered to be in parallel with the first parasitic capacitance C1 to ground, and the second negative capacitor circuit C20 may be considered to be in parallel with the second parasitic capacitance C2 to ground. The first negative capacitor circuit C10 and the second negative capacitor circuit C20 respectively cancel outs the parasitic capacitance at two input terminals of the differential amplifier, thereby reducing capacitance values at the two input terminals of the differential amplifier and achieving an increase in input impedance. In some embodiments, when equivalent capacitance values of the first negative capacitor circuit C10 and the second negative capacitor circuit C20 are expressed as the following formulas:

$$C10 = -C1 \; . \quad (4)$$

$$C20 = -C2 \; . \quad (5)$$

then the total capacitance to ground for both the first lead L1 and the second lead L2 is zero. In this scenario, the parasitic capacitance to ground at the input terminals of the differential amplifier is completely canceled out, significantly increasing the overall input impedance of the signal acquisition circuit and enhancing the signal acquisition circuit's resistance to power frequency interference.

In some embodiments, considering that the parasitic capacitance may slightly vary with lead movement in practical working environments, an absolute value of the equivalent capacitance of the negative capacitor circuit may not be exactly equal to the parasitic capacitance. In some embodiments, to better cancel out the parasitic capacitance at the input terminals of the differential amplifier, a relative error between the absolute value of an equivalent capacitance value of the first negative capacitor circuit C10 and the parasitic capacitance value C1 of the first lead L1 may be less than 50%, and a relative error between the absolute value of the equivalent capacitance value of the second negative capacitor circuit C20 and the parasitic capacitance value C2 of the second lead L2 may also be less than 50%. In some embodiments, to achieve a larger input impedance at the input terminals of the differential amplifier, the relative error between the absolute value of the equivalent capacitance value of the first negative capacitor circuit C10 and the parasitic capacitance value C1 of the first lead L1 may be less than 30%, and the relative error between the absolute value of the equivalent capacitance value of the second negative capacitor circuit C20 and the parasitic capacitance value C2 of the second lead L2 may also be less than 30%.

It should be understood that in practical applications, the smaller the relative error between the absolute value of the equivalent capacitance value of the first negative capacitor circuit C10 and the value of the first parasitic capacitance C1 to ground of the first lead L1, the more the first parasitic capacitance C1 is canceled out by the first negative capacitor circuit C10, leading to a larger input impedance for the entire signal acquisition circuit and, consequently, better signal acquisition results. The same principle applies to the second negative capacitor circuit C20, and further elaboration is not necessary here.

In some embodiments, the relative error between the absolute value of the equivalent capacitance value of the first negative capacitor circuit C10 and the value of the first parasitic capacitance C1 to ground of the first lead L1 refers to a ratio of a difference between the absolute value of the equivalent capacitance value of the first negative capacitance circuit C10 and the value of the parasitic capacitance to ground of the first lead L1 to the value of the parasitic capacitance to ground of the first lead L1.

In some embodiments, the equivalent input impedance $Z_{in}1$ of the first input terminal A of the differential amplifier 230 may be equivalent to a capacitor $R_{in}1$ and a capacitor $C_{in}1$ connected in parallel, and the equivalent input impedance $Z_{in}2$ of the second input terminal B of the differential amplifier 230 may be equivalent to a capacitor $R_{in2}$ and a capacitor $C_{in}2$ connected in parallel. In some embodiments, to further improve the input impedance performance of the signal acquisition circuit 200, the first negative capacitor circuit C10 and the second negative capacitor circuit C20 may be adjusted to further cancel out the influence of the equivalent input capacitances of the differential amplifier 230. In some embodiments, the equivalent capacitance values of the first negative capacitor circuit C10 and the second negative capacitor circuit C20 may be adjusted as follows:

$$C10 = -(C1 + C_{in}1) \; . \quad (6)$$

$$C20 = -(C2 + C_{in}2) \quad . \quad (7)$$

In some embodiments, to better cancel out the equivalent input capacitances at the input terminals of the differential amplifier, a relative error between the absolute value of the equivalent capacitance value of the first negative capacitor circuit C10 and a sum of a value of the parasitic capacitance C1 of the first lead L1 and a value of the equivalent input capacitance $C_{in}1$ of the first input terminal A of the differential amplifier 230 may be less than 50%; a relative error between the absolute value of the equivalent capacitance value of the second negative capacitor circuit C20 and a sum of a value of the parasitic capacitance C2 of the second lead L2 and a value of the equivalent input capacitance $C_{in}2$ of the second input terminal B of the differential amplifier 230 may also be less than 50%. In some embodiments, to achieve a larger input impedance at the input terminals of the differential amplifier, the relative error between the absolute value of the equivalent capacitance value of the first negative capacitor circuit C10 and the sum of the value of the parasitic capacitance C1 of the first lead L1 and the value of the equivalent input capacitance $C_{in}1$ of the first input terminal A of the differential amplifier 230 may be less than 30%; the relative error between the absolute value of the equivalent capacitance value of the second negative capacitor circuit C20 and the sum of the value of the parasitic capacitance C2 of the second lead L2 and the value of the equivalent input capacitance $C_{in}2$ of the second input terminal B of the differential amplifier 230 may also be less than 30%.

It should be understood that in practical applications, the smaller the relative error between the absolute value of the equivalent capacitance value of the first negative capacitor circuit C10 and the sum of the value of the parasitic capacitance C1 of the first lead L1 and the value of the equivalent input capacitance $C_{in}1$ of the first input terminal A of the differential amplifier 230, the more the parasitic capacitance C1 is canceled out by the first negative capacitor circuit C10, leading to a larger input impedance for the entire signal acquisition circuit and, consequently, better signal acquisition results. The same principle applies to the second negative capacitor circuit C20, and further elaboration is not necessary here.

As a result, the first negative capacitor circuit C10 and the second negative capacitor circuit C20 respectively cancel out the parasitic capacitance to ground of the leads (L1 and L2) and the equivalent input capacitance ($C_{in}1$ and $C_{in}2$) of the corresponding input terminals (A and B) of the differential amplifier 230, thereby further increasing the overall input impedance of the signal acquisition circuit.

In some embodiments, the signal acquisition circuit 200 may further include a third negative capacitor circuit C30. The third negative capacitor circuit C30 may be electrically connected between the first lead L1 and the second lead L2, and is parallel to the lead-to-lead parasitic capacitance C3. In some embodiments, due to the lower impact of the lead-to-lead parasitic capacitance C3 on circuit performance, the signal acquisition circuit 200 may not need to set the third negative capacitor circuit C30. However, in cases where a higher performance is required for the signal acquisition circuit 200, the third negative capacitor circuit C30 may be included. A value of the third negative capacitor circuit C30 may be represented by the following formula:

$$C30 = -C3 \quad . \quad (8)$$

In some embodiments, a relative error between an absolute value of an equivalent capacitance of the third negative capacitor circuit C30 and a value of the parasitic capacitance C3 between the two leads (L1 and L2) may be less than 50%. In some embodiments, the relative error between the absolute value of the equivalent capacitance of the third negative capacitor circuit C30 and the value of the parasitic capacitance C3 between the two leads (L1 and L2) may be less than 30%. It should be understood that in practical applications, the smaller the relative error between the absolute value of the equivalent capacitance of the third negative capacitor circuit C30 and the value of the parasitic capacitance C3 between the two leads, the more the third negative capacitor circuit C30 cancel outs the lead-to-lead parasitic capacitance C3, leading to a larger input impedance for the entire signal acquisition circuit and, consequently, better signal acquisition results.

In some embodiments, the signal acquisition circuit may also include a feedback control circuit to adjust the equivalent capacitance values of the first negative capacitor circuit C10 and the second negative capacitor circuit C20. For example, the adjustment may be achieved by changing resistance values in the negative capacitor circuits.

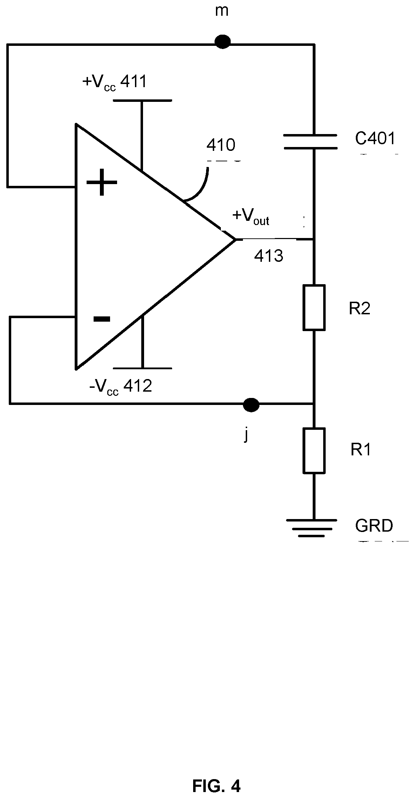

Regarding the specific structure of the first negative capacitor circuit C10, the second negative capacitor circuit C20, and the third negative capacitor circuit C30, please refer to the relevant content in FIG. 4.

FIG. 4 is a schematic diagram of a structure of negative capacitor circuits in the signal acquisition circuit 200 according to some embodiments of the present disclosure.

In some embodiments, the first negative capacitor circuit C10 may have the structure shown in FIG. 4. As illustrated in FIG. 4, the first negative capacitor circuit C10 may include a first operational amplifier 410, a first resistor R1, a second resistor R2, and a first capacitor C401. In some embodiments, a reverse input terminal of the first operational amplifier 410 may be connected to ground through the first resistor R1, the reverse input terminal of the first operational amplifier 410 may be connected to an output terminal of the first operational amplifier through the second resistor R2, and a forward input terminal of the first operational amplifier 410 may be connected to the output terminal of the first operational amplifier through the first capacitor C401. In some embodiments, point m in FIG. 4 and point a in FIG. 3 are points at the same potential, and a lead may be used to connect points a and m electrically.

In some embodiments, the second negative capacitor circuit C20 may also have the structure shown in FIG. 4. In this case, points m in FIG. 4 and b in FIG. 3 are points at the same potential, and leads may be used to connect points m and b electrically.

It should be noted that the negative capacitor circuit shown in FIG. 4 is an equivalent circuit with a negative capacitance effect. Specifically, for the negative capacitor circuit shown in FIG. 4, the equivalent impedance of this negative capacitor circuit is the impedance between points m and GND. Assuming the impedance between points m and GND is denoted as $z_a$, the impedance $z_a$ may be represented by the following formula (9):

$$z_a = \frac{R_1}{j\omega\,(-C_{401})\,R_2} \qquad (9)$$

In other words, point m and GND may be equivalent to including a negative capacitance or generated by a negative capacitance. Assuming the negative capacitance between point m and GND is denoted as $C_a$, the equivalent capacitance value of $C_a$ may be represented by the following formula (10):

$$C_a = \frac{-C_{401} R_2}{R_1} \qquad (10)$$

It may be understood that $C_a$ is the equivalent capacitance of the negative capacitor circuit shown in FIG. 4.

In some embodiments, the first resistor R1 and the second resistor R2 may be variable resistors (e.g., potentiometers, resistor boxes, and potentiometers) with adjustable resistance values or resistors with fixed values. In some embodiments, resistance values of the first resistor R1 and the second resistor R2 may be equal or unequal. It may be understood that in some embodiments, a magnitude of the equivalent capacitance $C_a$ may be adjusted by adjusting the resistance values of the first resistor R1 and the second resistor R2.

In some embodiments, the first capacitor C401 may be a paper dielectric capacitor, a metallized paper dielectric capacitor, a ceramic capacitor, a thin film capacitor, an oil-impregnated paper dielectric capacitor, an aluminum electrolytic capacitor, a semi-variable capacitor, a variable capacitor, etc. In some embodiments, the magnitude of the equivalent capacitance $C_a$ may be adjusted by adjusting a capacitance value of the first capacitor C401.

In some embodiments, the operational amplifier 410 may be powered by a dual power supply, for example, voltage $+V_{cc}$ (411) as a positive power supply and voltage $-V_{cc}$ (412) as a negative power supply, with an amplified signal generated by the operational amplifier 410 being outputted at an output terminal 413. In some embodiments, the operational amplifier 410 may also be powered by a single power supply, details of which is not elaborated here.

In some embodiments, the first negative capacitor circuit C10 and the second negative capacitor circuit C20 may also have structures other than the structure shown in FIG. 4.

In some embodiments, the signal acquisition circuit 200 further includes a third negative capacitor circuit C30. The third negative capacitor circuit C30 is electrically connected between the first lead L1 and the second lead L2 and has a negative capacitance effect.

In some embodiments, the third negative capacitor circuit C30 may have the structure shown in FIG. 4. In this case, point m in FIG. 4 is at the same potential as point c in FIG. 3, and GND in FIG. 4 is at the same potential as point d in FIG. 3. Leads may be used to electrically connect point c in FIG. 3 to point m in FIG. 4 and point d in FIG. 3 to GND in FIG. 4.

In some embodiments, the third negative capacitor circuit C30 may also have structures other than the structure shown in FIG. 4, as long as it may cancel out the lead-to-lead parasitic capacitance C3.

It should be noted that the description of the signal acquisition circuit 100, the signal acquisition circuit 200, and the structures thereof provided above is for convenience of description and does not limit the scope of the present disclosure to the embodiments mentioned.

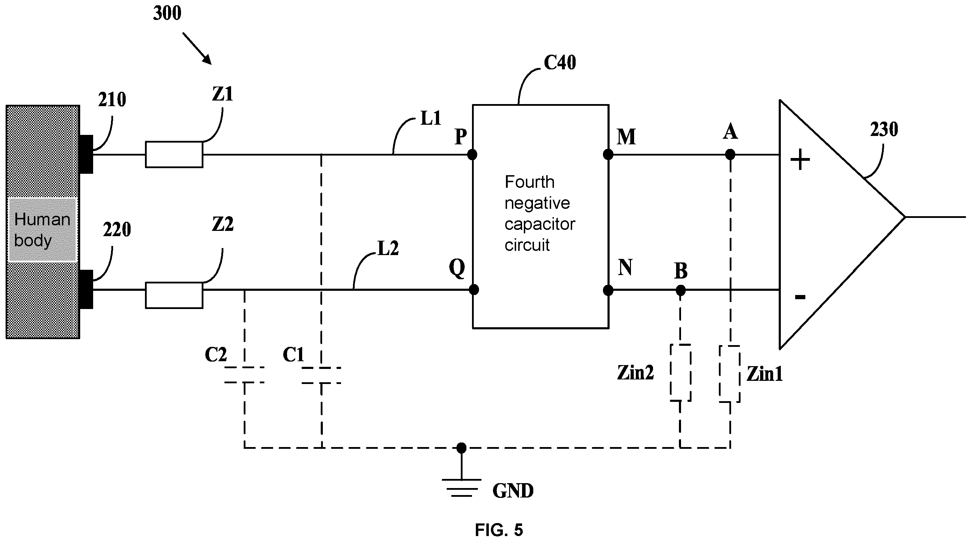

FIG. 5 is a schematic diagram of a signal acquisition circuit 300 according to some embodiments of the present disclosure.

As shown in FIG. 5, the signal acquisition circuit 300 may include a first electrode 210, a second electrode 220, a differential amplifier 230, a first lead L1, a second lead L2, and a fourth negative capacitor circuit C40. For relevant descriptions of the first electrode 210, the second electrode 220, the differential amplifier 230, the first lead L1, and the second lead L2, please refer to FIG.s 1-4 and the corresponding descriptions thereof, which will not be reiterated here.

Combining the descriptions provided in the previous embodiments, there is a first parasitic capacitance C1 between the first lead L1 and the ground, and a second parasitic capacitance C2 between the second lead L2 and the ground. Due to the parallel connection of the first parasitic capacitance C1 with the ground impedance $Z_{in}1$ and the second parasitic capacitance C2 with the ground impedance $Z_{in}2$ of the differential amplifier, the overall input impedance of the circuit is significantly reduced. This exacerbates the impact of power frequency interference on signal acquisition.

Therefore, in some embodiments, to reduce the impact of parasitic capacitance and increase the input impedance of the differential amplifier 230, the signal acquisition circuit 300 may be provided with a fourth negative capacitor circuit C40. In some embodiments, the first electrode 210 may be electrically connected to a first input terminal P of the fourth negative capacitor circuit C40 through the first lead L1, and the second electrode 220 may be electrically connected to a second input terminal Q of the fourth negative capacitor circuit C40 through the second lead L2. A first output terminal M of the fourth negative capacitor circuit C40 may be connected to the first input terminal A of the differential amplifier 230, and a second output terminal N of the fourth negative capacitor circuit C40 may be connected to the second input terminal B of the differential amplifier 230. The fourth negative capacitor circuit C40 has a negative capacitance effect, which may cancel out parasitic capacitances to the ground of the leads, reducing the impact of the parasitic capacitances on the input impedance of the circuit and signal acquisition, thereby further improving the performance of the signal acquisition circuit and the effectiveness of signal acquisition.

In some embodiments, the negative capacitor circuit C40 may be implemented using an amplifier with a fixed gain and a feedback capacitor, thereby cancel outing both the first parasitic capacitor C1 to ground and the second parasitic capacitor C2 to ground. Specific implementation details of the negative capacitor circuit C40 may be found in FIG.s 6A and 6B and the relevant descriptions thereof.

] In some embodiments, the signal acquisition circuit 300 may further include a third negative capacitor circuit C30. The third negative capacitor circuit C30 may be electrically connected between the first lead L1 and the second lead L2 and connected in parallel with the lead-to-lead parasitic capacitance C3. In some embodiments, since the presence of the lead-to-lead parasitic capacitance C3 has a relatively low impact on the circuit performance, the signal acquisition circuit 200 may not need to be provided with the third negative capacitor circuit C30. In some embodiments, when higher requirements are placed on the signal acquisition circuit 200, the signal acquisition circuit 200 may be provided with the third negative capacitor circuit C30. More implementation details of the third negative capacitor circuit C30 may be found in FIG.s 3 and 4 and the relevant descriptions thereof, which will not be reiterated here.

In some embodiments, the signal acquisition circuit may further include a feedback control circuit configured to adjust an equivalent capacitance value of the fourth negative capacitor circuit C40, for example, by changing the resistance or capacitance values in the negative capacitor circuit.

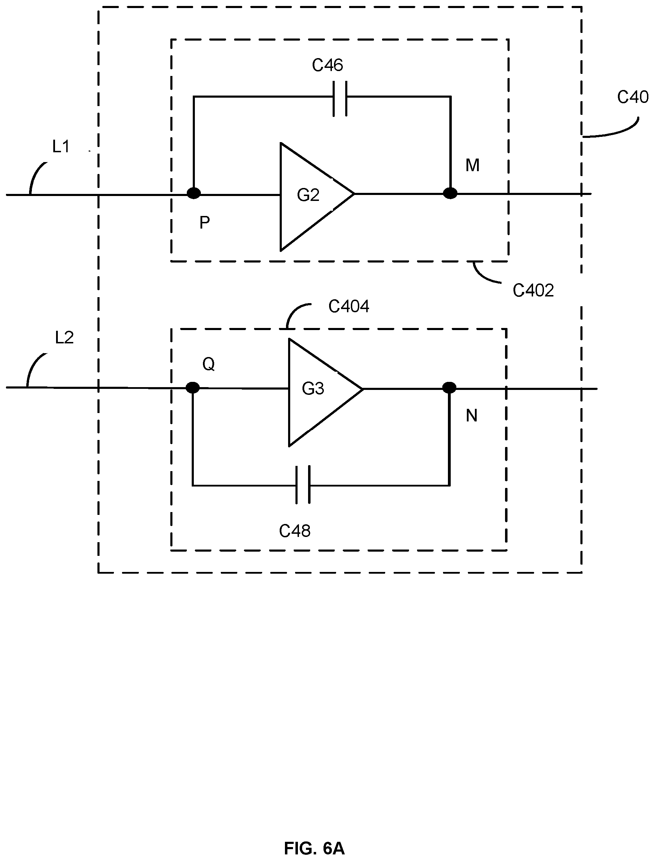

FIG. 6A is a structural schematic diagram of the fourth negative capacitor circuit C40 in the signal acquisition circuit 300 according to some embodiments of the present disclosure.

In some embodiments, the fourth negative capacitor circuit C40 may include a first unit C402 and a second unit C404. The first unit C402 may include a first amplifier G2 and a third negative feedback capacitor C46, and the second unit C404 may include a second amplifier G3 and a fourth negative feedback capacitor C48. In some embodiments, both the first amplifier G2 and the second amplifier G3 are fixed-gain amplifiers.

In some embodiments, the input terminal of the first amplifier G2 is the first input terminal P of the fourth negative capacitor circuit C40, and the input terminal of the second amplifier G3 is the second input terminal Q of the fourth negative capacitor circuit C40. Similarly, the output terminal of the first amplifier G2 is the first output terminal M of the fourth negative capacitor circuit C40, and the output terminal of the second amplifier G3 is the second output terminal N of the fourth negative capacitor circuit C40.

In some embodiments, the third negative feedback capacitor C46 may be connected in series between the input terminal P of the fourth negative capacitor circuit C40 and the output terminal M of the first amplifier G2, and the fourth negative feedback capacitor C48 may be connected in series between the input terminal Q of the fourth negative capacitor circuit C40 and the output terminal N of the second amplifier G3.

In some embodiments, if the gain of the first amplifier G2 is constant and denoted as g1, then the gain of the second amplifier G3 is also constant at g1. The equivalent capacitance value of the first unit C402 of the fourth negative capacitor circuit C40 may be represented by the following formula:

$$C402 = -C46(g1 - 1) \ . \quad (11)$$

The equivalent capacitance value of the second unit C404 of the fourth negative capacitor circuit C40 may be represented by the following formula:

$$C404 = -C48(g1 - 1). \quad (12)$$

In some embodiments, the first amplifier G2 and the second amplifier G3 may be amplifiers with an equal and fixed gain. For example, if the gain of the first amplifier G2 is constant and denoted as g1, then the gain of the second amplifier G3 is also constant at g1. In some embodiments, the gain of the first amplifier G2 and the second amplifier G3 may range from 0 to 100 dB, with a preferred range of 0 to 10 dB.

In some embodiments, values of the third negative feedback capacitor C46 and the fourth negative feedback capacitor C48 are equal. Thus, the first unit C402 and the second unit C404 of the fourth negative capacitor circuit C40 form a highly symmetric structure, enabling the signal acquisition circuit 300 to achieve a larger common-mode rejection ratio. In some embodiments, if the gain of the first amplifier G2 is not equal to the gain of the second amplifier G3, the common-mode rejection ratio of the circuit decreases, and a desired reduction in power frequency interference may not be achieved.

In some embodiments, each of the first amplifier G2 and the second amplifier G3 may be composed of a plurality of amplifiers cascaded with a fixed gain.

In some embodiments, the first unit C402 of the fourth negative capacitor circuit C40 may be used to cancel out the first parasitic capacitance C1 to ground, and the second unit C404 of the fourth negative capacitor circuit C40 may be used to cancel out the second parasitic capacitance C2 to ground. Thus, the fourth negative capacitor circuit C40 may be used to simultaneously cancel out both the first parasitic capacitance C1 to ground and the second parasitic capacitance C2 to ground. In some embodiments, the equivalent capacitance values of the first unit C402 and the second unit C404 may be represented by the following formulas:

$$C402 = -C1 \ . \quad (13)$$

$$C404 = -C2 \ . \quad (14)$$

As a result, total capacitances to ground of the first lead L1 and the second lead L2 are both zero. At this point, the parasitic capacitances to ground at the input terminals of the differential amplifier 230 are completely canceled out, and the overall input impedance of the signal acquisition circuit 300 is significantly increased. Therefore, the resistance to power frequency interference is enhanced.

In some embodiments, considering the possibility of slight variations in parasitic capacitance due to lead movement in practical working environments, a relative error between an absolute value of the equivalent capacitance value of the first unit C402 of the fourth negative capacitor circuit C40 and the value of the parasitic capacitance C1 of the first lead L1 may be less than 50%. Similarly, a relative error between an absolute value of the equivalent capacitance value of the second unit C404 of the fourth negative capacitor circuit C40 and the value of the parasitic capacitance C2 of the second lead L2 may be less than 50%. In some embodiments, the relative error between the absolute value of the equivalent capacitance value of the first unit C402 of the fourth negative capacitor circuit C40 and the value of the parasitic capacitance C1 of the first lead L1 may be less than 30%. Similarly, the relative error between the absolute value of the equivalent capacitance value of the second unit C404 of the fourth negative capacitor circuit C40 and the value of the parasitic capacitance C2 of the second lead L2 may be less than 30%.

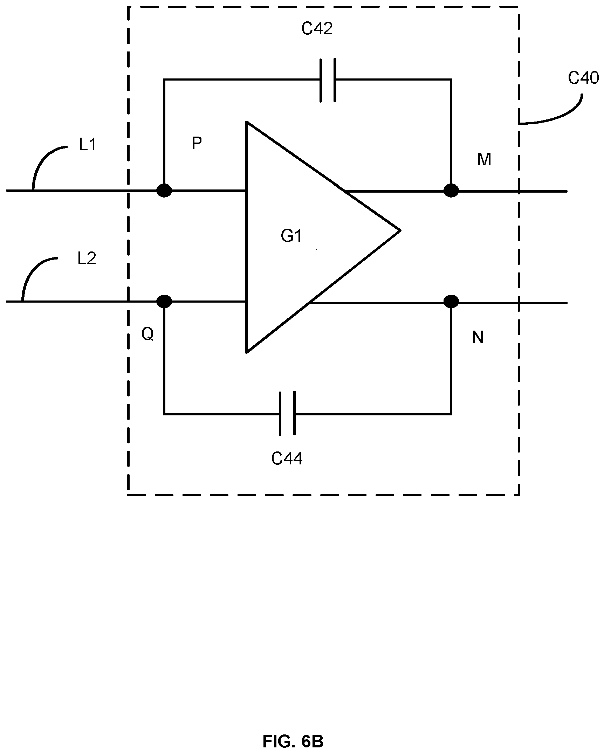

FIG. 6B is another structural schematic diagram of the fourth negative capacitor circuit C40 in the signal acquisition circuit 300 according to some embodiments of the present disclosure.

In some embodiments, the fourth negative capacitor circuit C40 may include a double-ended differential amplifier G1, a first negative feedback capacitor C42, and a second negative feedback capacitor C44.

In some embodiments, the first input terminal of the double-ended differential amplifier G1 is the first input terminal P of the fourth negative capacitor circuit C40, and the second input terminal of the double-ended differential amplifier G1 is the second input terminal Q of the fourth negative capacitor circuit C40. Similarly, the first output terminal of the double-ended differential amplifier G1 is the first output terminal M of the fourth negative capacitor circuit C40, and the second output terminal of the double-ended differential amplifier G1 is the second output terminal N of the fourth negative capacitor circuit C40. The first input terminal P and the first output terminal M of the double-ended differential amplifier G1 may be connected in series through the first negative feedback capacitor C42, and the second input terminal Q and the second output terminal N of the double-ended differential amplifier G1 may be connected in series through the second negative feedback capacitor C44.

In some embodiments, the double-ended differential amplifier G1 may be a fixed-gain amplifier. In some embodiments, the gain g0 of the double-ended differential amplifier G1 may range from 0 to 100 dB, preferably within the range of 0 to 10 dB.

In some embodiments, the fourth negative capacitor circuit C40 may be used to simultaneously cancel out the first parasitic capacitance C1 to ground and the second parasitic capacitance C2 to ground. In some embodiments, the double-ended differential amplifier G1 may be composed of a plurality of fixed-gain amplifiers. For example, the double-ended differential amplifier G1 may be comprised of two amplifiers, G11 and G12, with a fixed gain g0, wherein both the amplifiers G11 and G12 have an identical and fixed gain, and their structures are also identical. In some embodiments, an input terminal of the amplifier G11 is point P, and an output terminal of the amplifier G11 is point M. The first negative feedback capacitor C42 may be connected in series between points P and M. The circuit structure formed by the amplifier G11 and the first negative feedback capacitor C42 may be used to cancel out the first parasitic capacitance C1 to ground. In some embodiments, an input of the amplifier G12 is point Q, and an output of the amplifier G12 is point N. The second negative feedback capacitor C44 may be connected in series between points Q and N. The circuit structure formed by the amplifier G12 and the second negative feedback capacitor C44 may be used to cancel out the second parasitic capacitance C2 to ground. In some embodiments, an equivalent capacitance value C11 for the circuit structure formed by the amplifier G11 and the first negative feedback capacitor C42 and an equivalent capacitance value C12 for the circuit structure formed by the amplifier G12 and the second negative feedback capacitor C44 may be represented by the following formulas:

$$C11 = -C42(g0 - 1) \ . \quad (15)$$

$$C12 = -C45(g0 - 1) \ . \quad (16)$$

At this point, the circuit structure formed by the amplifier G11 and the first negative feedback capacitor C42 may be used to cancel out the first parasitic capacitance C1 to ground, and the circuit structure formed by the amplifier G12 and the second negative feedback capacitor C44 may be used to cancel out the second parasitic capacitance C2 to ground. In this configuration, the parasitic capacitances to ground at the input terminals of the differential amplifier may be canceled out, significantly increasing the overall input impedance of the signal acquisition circuit and enhancing its resistance to power frequency interference.

In some embodiments, to better cancel out the parasitic capacitances to ground at the input terminals of the differential amplifier, a relative error between the absolute value of the value of the equivalent capacitance C11 of the circuit structure formed by the amplifier G11 and the first negative feedback capacitor C42 and the value of the parasitic capacitance C1 to ground at the first lead L1 may be less than 50%; a relative error between the absolute value of the value of the equivalent capacitance C12 of the circuit structure formed by the amplifier G12 and the second negative feedback capacitor C44 and the value of the parasitic capacitance C2 to ground at the second lead L2 may be less than 50%. In some embodiments, to increase the input impedance of the differential amplifier, the relative error between the absolute value of the value of the equivalent capacitance C11 of the circuit structure formed by the amplifier G11 and the first negative feedback capacitor C42 and the value of the parasitic capacitance C1 to ground at the first lead L1 may be less than 30%; the relative error between the absolute value of the value of the equivalent capacitance C12 of the circuit structure formed by the amplifier G12 and the second negative feedback capacitor C44 and the value of the parasitic capacitance C2 to ground at the second lead L2 may be less than 30%.

It should be understood that in practical applications, the smaller the relative error between the absolute value of the value of the equivalent capacitance C11 of the circuit structure formed by the amplifier G11 and the first negative feedback capacitor C42 and the value of the parasitic capacitance C1 to ground at the first lead L1, and the smaller the relative error between the absolute value of the value of the equivalent capacitance C12 of the circuit structure formed by the amplifier G12 and the second negative feedback capacitor C44 and the value of the parasitic capacitance C2 to ground at the second lead L2, the more the fourth negative capacitor circuit C40 cancel outs the parasitic capacitances C1 and C2, resulting in a larger overall input impedance of the signal acquisition circuit, and consequently, improved signal acquisition performance.

In some embodiments, the fourth negative capacitor circuit C40 may have other structures than the structures illustrated in FIG.s 6A and 6B, as long as the equivalent capacitance value of the fourth negative capacitor circuit C40 may cancel out the parasitic capacitance C1 at the first lead L1 and the parasitic capacitance C2 at the second lead L2, without imposing excessive limitations.

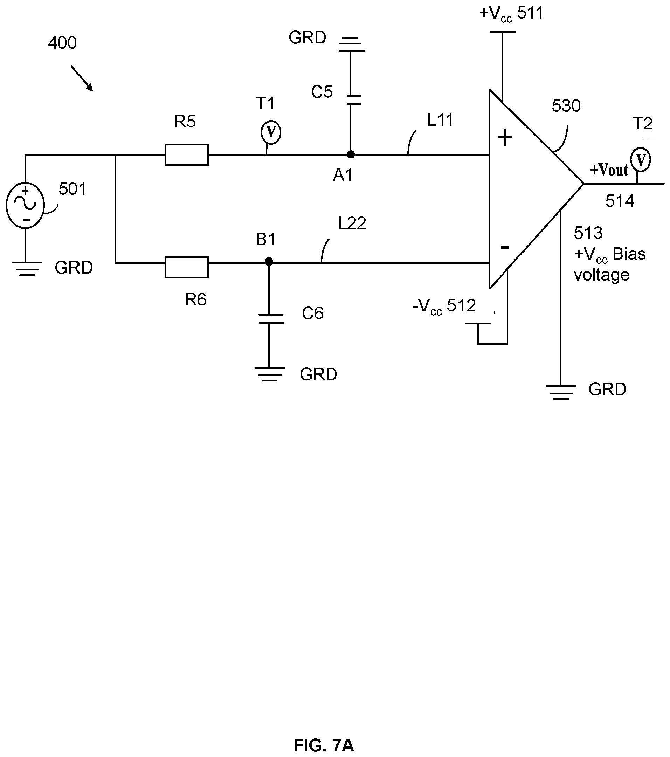

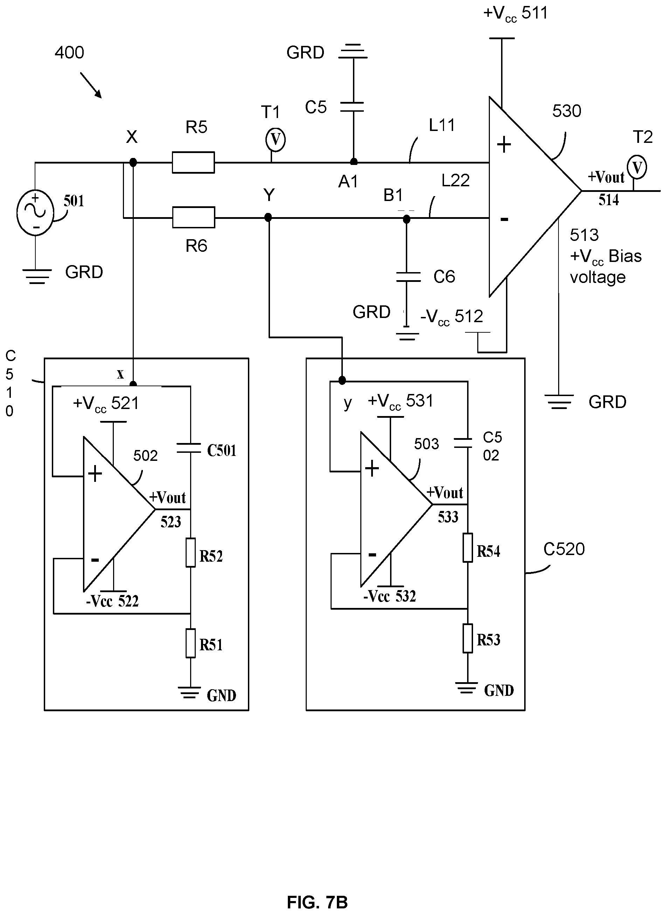

FIG. 7A is a schematic diagram illustrating an effect circuit before eliminating power frequency interference according to some embodiments of the present disclosure. FIG. 7B is a schematic diagram illustrating an effect circuit after eliminating power frequency interference according to some embodiments of the present disclosure.

In some embodiments, as shown in FIG. 7A, a simulated signal acquisition circuit 400 is provided. The signal acquisition circuit 400 includes a common-mode power frequency source 501, a differential amplifier 530, a resistor R5 with a resistance of 10MΩ, a resistor R6 with a resistance of 100KΩ, and capacitors C5 and C6, each with a capacitance of 6pF. The resistor R5 is connected to a first lead L11 and a first input terminal (A1) of the differential amplifier 530, and the resistor R6 is connected to a second lead L22 and a second input terminal (B1) of the differential amplifier 530. One end of the capacitor C5 is connected to the first input terminal (A1) of the differential amplifier 530, and also connected to the first lead L11, while another end of the capacitor C5 is grounded. One end of the capacitor C6 is connected to the second input terminal (B1) of the differential amplifier 530, and also connected to the second lead L22, while another end of capacitor C6 is grounded.

It should be noted that the common-mode power frequency source 501 is an AC power source with a voltage peak value of 300mV and a frequency of 50Hz, simulating a common-mode power frequency signal generated by a human body.

The resistances of R5 and R6 may be different to simulate impedance mismatch formed between two electrodes when a wearable device collects signals from the human body in. The capacitors C5 and C6 may be used to simulate parasitic capacitances formed by the two leads (L11and L22) to ground when the wearable device collects signals from the human body.

In some embodiments, the differential amplifier 530 may powered differentially on two ends, where a first power supply terminal (511) of the differential amplifier 530 may connect to a positive power supply +$V_{cc}$, a second power supply terminal (512) may connect to a negative power supply -$V_{cc}$, and a bias voltage receiving end (513) may be grounded. In some embodiments, the positive power supply +$V_{cc}$ may provide a voltage of 3.3V, and the negative power supply -$V_{cc}$ may provide a voltage of -3.3V.

In some embodiments, the signal acquisition circuit 400 may further include a first probe T1 and a second probe T2. The first probe T1 may be positioned on a signal input side and connect to the first input terminal (A1) of the differential amplifier 530. The second probe T2 may be positioned on a signal output side and connect to the output terminal (514) of the differential amplifier 530. The first probe T1 and the second probe T2 may be used to measure voltage peak values, effective voltage values, frequencies, etc., of a voltage signal before and after the voltage signal is input to the differential amplifier 530. After testing, the obtained data is presented in Table 1:

Table 1

|  | Peak voltage | Effective voltage | DC | Voltage frequency |
|---|---|---|---|---|
| First probe T1 | 11.4mV | 196mV | 196mV | 50Hz |
| Second probe T2 | 11.4mV | 195mV | 195mV | 50.2Hz |

FIG. 7B is a schematic diagram illustrating an effect circuit after eliminating power frequency interference according to some embodiments of the present disclosure. In FIG. 7B, negative capacitor circuits C510 and C520 are added on the basis of FIG. 7A. The negative capacitor circuits C510 and C520 both adopt the structure shown in FIG. 4, where a point x in the negative capacitor circuit C510 is electrically connected to a point X on the first lead L11, and a point y in the negative capacitor circuit C520 is electrically connected to a point Y on the second lead L22.

In some embodiments, a first power supply terminal (521) of an operational amplifier 502 of the negative capacitor circuit C510 is connected to a positive power supply +$V_{cc}$, a second power supply terminal (522) of the operational amplifier 502 is connected to a negative power supply -$V_{cc}$, and an output terminal (523) of the operational amplifier 502 outputs an amplified voltage. In some embodiments, the positive power supply +$V_{cc}$ provides a voltage of 3.3V, the negative power supply -$V_{cc}$ provides a voltage of -3.3V, and the capacitance of a capacitor C501 is 6pF, while the resistances of resistors R51 and R52 are both 10K$\Omega$.

According to the formula (10) mentioned above, the capacitance value of the negative capacitor circuit C510 may be calculated as -6pF.

The negative capacitor circuit C520 adopts the same structure and components of the same values as negative capacitor circuit C510, and therefore, the capacitance value of the negative capacitor circuit C520 is also -6pF.

To verify that the addition of the two negative capacitor circuits indeed reduces the power frequency interference in the entire signal acquisition circuit 400, testing is performed again using a first probe T1 and a second probe T2. The obtained data is presented in Table 2:

Table 2

|  | Peak voltage | Effective voltage | DC | Voltage frequency |
|---|---|---|---|---|
| First probe T1 | 1.17mV | 196mV | 196mV | 50Hz |
| Second probe T2 | 1.10mV | 195mV | 195mV | 50.2Hz |

Comparing the data in Tables 1 and 2, it may be observed that before adding the negative capacitor circuits C510 and C520, the peak voltages measured by the first probe T1 and the second probe T2 were both 11.4mV. After adding the negative capacitor circuits C510 and C520, the peak voltage measured by the first probe T1 is 1.17mV, and the peak voltage measured by the second probe T2 is 1.10mV. Thus, it may be concluded that by adding the negative capacitor circuits C510 and C520, the power frequency interference in the entire circuit is reduced by more than 10 times.

This result indicates that by introducing negative capacitor circuits at the input of the differential amplifier, the input impedance may be significantly increased, thereby reducing the interference caused by electrode impedance mismatch, and thus greatly reducing the risk of circuit saturation.

In some embodiments, the signal acquisition circuits 200 and 300 described above may be applied to a wearable device. The wearable device (e.g., a piece of clothing, a wristband, a shoulder strap, etc.) may be positioned on various parts of the body (e.g., a calf, a thigh, the waist, the back, the chest, a shoulder, the neck, etc.) of a user to collect physiological signals from different parts of the body of the user in various states. Subsequently, the collected signals may be further processed.

It should be noted that the above signal acquisition circuits may be used in scenarios requiring the detection of signals that reflect the user's body state. For example, the physiological signals may include respiratory signals, electrocardiogram signals, electromyogram signals, electroencephalogram signals, blood pressure signals, temperature signals, etc., or any combinations thereof. In some embodiments, the wearable device that collect the physiological signals may be applied in emerging cross-industries such as medical, gaming and entertainment, health education, etc. For example, the wearable device that collect the physiological signals may be combined with technologies like virtual reality (VR) and electromyography (EMG) collection to promote the development of immersive entertainment and education, or combined with technologies like mechatronics and exoskeleton machines to reduce medical costs and promote healthcare development. The specific application scenarios of the signal acquisition circuit and the wearable device are not limited by the present disclosure.

The potential beneficial effects of the embodiments of the present disclosure may include but are not limited to: effectively increasing the input impedance of the entire signal acquisition circuit by configuring the negative capacitor circuit to cancel out the parasitic capacitance in the signal acquisition circuit, thereby significantly reducing the interference of parasitic capacitance on the entire signal acquisition circuit and improving the effectiveness of signal acquisition.

It should be noted that the beneficial effects that may arise from different embodiments may vary. In different embodiments, the potential beneficial effects may be a combination of any of the mentioned effects or any other possible beneficial effects that may be achieved.

The basic concepts have been described above, and it is apparent that to a person skilled in the art, the above detailed disclosure is intended as an example only and does not constitute limitations of the present disclosure.

Also, the present disclosure uses specific words to describe the embodiments of the present disclosure. For example, "an embodiment," "one embodiment," and/or "some embodiments" are meant to refer to a certain feature, structure, or characteristic associated with at least one embodiment of the present disclosure. Accordingly, it should be emphasized and noted that "an embodiment" or "one embodiment" or "an alternative embodiment" mentioned two or more times in different places in the present disclosure do not necessarily refer to the same embodiment. Furthermore, certain features, structures, or characteristics in one or more embodiments of the present disclosure may be suitably combined.

Additionally, those skilled in the art may understand that various aspects of the present disclosure may be explained and described through several patentable categories or situations, including any new and useful combination of processes, machines, products, or substances, or any new and useful improvements thereof. Accordingly, various aspects of the present disclosure may be entirely implemented by hardware, entirely by software (including firmware, resident software, microcode, etc.), or a combination of hardware and software. The terms "data block," "module," "engine," "unit," "component," or "system" may be used to refer to the aforementioned hardware or software.

The computer storage medium may include a computer product containing computer program encoding. The computer storage medium may contain a propagated data signal including computer program encoding, for example, on baseband or as part of a carrier. The propagated signal may take various forms, including electromagnetic forms, optical forms, or suitable combinations thereof. The computer storage medium may be any computer-readable medium other than computer-readable storage media that may communicate, propagate, or transmit programs for use by or in connection with an instruction execution system, apparatus, or device. The program encoding on the computer storage medium may be propagated through any suitable medium, including radio, cable, fiber-optic cables, radiofrequency (RF), or the like, or a combination thereof.

Furthermore, unless explicitly stated in the claims, the use of order, numbers, letters, or other names for processing elements and sequences is not intended to limit the order of the processes and methods of the present disclosure. While various examples have been discussed in the disclosure as currently considered useful embodiments of the invention, it should be understood that such details are provided for illustrative purposes only. The appended claims are not limited to the disclosed embodiments. For example, although the described system components may be implemented through a hardware device, they may also be realized solely through a software solution, such as installing the described system on an existing processing or mobile device.

Similarly, it should be noted that, for the sake of simplifying the presentation of embodiments disclosed in the present disclosure and aiding in understanding one or more embodiments of the present disclosure, various features have been sometimes combined into a single embodiment, drawing, or description. However, this manner of disclosure does not imply that the features required by the claims are more than the features mentioned in the claims. In fact, the features of the embodiments are less than all the features of the single embodiment disclosed in the foregoing disclosure.

In some embodiments, numeric values describing the composition and quantity of attributes are used in the description. It should be understood that such numeric values used for describing embodiments may be modified with qualifying terms such as "about," "approximately" or "generally". Unless otherwise stated, "about," "approximately" or "generally" indicates that a variation of $\pm 20\%$ is permitted in the described numbers. Accordingly, in some embodiments, the numerical parameters used in the disclosure and claims are approximations, which can change depending on the desired characteristics of the individual embodiment. In some embodiments, the numerical parameters should take into account a specified number of valid digits and employ a general manner of bit retention. Although the numerical ranges and parameters used in some embodiments of the present disclosure to confirm the breadth of the range are approximations, in specific embodiments, such numerical values are set as precisely as practicable.

In closing, it should be understood that the embodiments described in the present disclosure are used only to illustrate the principles of the embodiments of the present disclosure. Other deformations may also fall within the scope of the present disclosure. Therefore, by way of example and not limitation, alternative configurations of the embodiments disclosed in the present disclosure may be considered consistent with the appended set of claims.

**Claims**

1. A signal acquisition circuit, comprising:

   a differential amplifier (130, 230, 530),
   a first electrode (110, 210) and a second electrode (120, 220), wherein the first electrode is connected to a first input terminal (A, A1) of the differential amplifier through a first lead (L1, L11), and the second electrode is connected to a second input terminal (B, B1) of the differential amplifier through a second lead (L2, L22);
   a first negative capacitance circuit (C10) and a second negative capacitance circuit (C20), wherein the first negative capacitance circuit is electrically connected between the first lead and ground, and the second negative capacitance circuit is electrically connected between the second lead and the ground, and both the first negative capacitance circuit and the second negative capacitance circuit exhibit a negative capacitance effect;
   the signal acquisition circuit is **characterized by** further comprising a third negative capacitance circuit (C30) electrically connected between the first lead and the second lead, wherein the third negative capacitance circuit exhibits a negative capacitance effect.

2. The signal acquisition circuit according to claim 1, wherein a relative error between an absolute value of an equivalent capacitance value of the first negative capacitance circuit and a value of a parasitic capacitance to ground of the first lead is less than 50%, and
   a relative error between an absolute value of an equivalent capacitance value of the second negative capacitance circuit and a value of a parasitic capacitance to ground of the second lead is less than 50%.

3. The signal acquisition circuit according to claim 1, wherein the first input terminal of the differential amplifier has a first equivalent input capacitance, and the second input terminal of the differential amplifier has a second equivalent input capacitance,

   a relative error between an absolute value of an equivalent capacitance value of the first negative capacitance circuit and a sum of a value of a parasitic capacitance to ground of the first lead and a value of the first equivalent input capacitance is less than 50%, and
   a relative error between an absolute value of an equivalent capacitance value of the second negative capacitance circuit and a sum of a value of a parasitic capacitance to ground of the second lead and a value of the second equivalent input capacitance is less than 50%.

4. The signal acquisition circuit according to claim 1, wherein a relative error between an absolute value of an equivalent capacitance of the third negative capacitance circuit and a value of a parasitic capacitance value between the first lead and the second lead is less than 50%.

5. The signal acquisition circuit according to claim 1, wherein the first negative capacitance circuit includes a first operational amplifier (410), a first resistor (R1), a second resistor (R2), and a first capacitor (C401), and the second negative capacitance circuit includes a second operational amplifier, a third resistor, a fourth resistor, and a second capacitor;

   a reverse input terminal of the first operational amplifier is connected to the ground through the first resistor, the reverse input terminal of the first operational amplifier is connected to an output terminal of the first operational amplifier through the second resistor, and a forward input terminal of the first operational amplifier is connected to the output terminal of the first operational amplifier through the first capacitor; and

a reverse input terminal of the second operational amplifier is connected to the ground through the third resistor, the reverse input terminal of the second operational amplifier is connected to an output terminal of the second operational amplifier through the fourth resistor, and a forward input terminal of the second operational amplifier is connected to the output terminal of the second operational amplifier through the second capacitor.

6. The signal acquisition circuit according to claim 5, wherein the forward input terminal of the first operational amplifier is connected to the first lead, and the forward input terminal of the second operational amplifier is connected to the second lead.

7. The signal acquisition circuit according to claim 1, wherein an common mode input impedance of the differential amplifier is greater than one hundred megaohms.

8. The signal acquisition circuit according to claim 1, wherein the differential amplifier is powered by a single power supply.

9. A signal acquisition circuit, comprising:

a differential amplifier,
a first electrode and a second electrode, and
a fourth negative capacitance circuit (C40), wherein the first electrode is connected to a first input terminal (P) of the fourth negative capacitance circuit through a first lead, the second electrode is connected to a second input terminal (Q) of the fourth negative capacitance circuit through a second lead, a first output terminal (M) of the fourth negative capacitance circuit is connected to the first input terminal of the differential amplifier, and a second output terminal (N) of the fourth negative capacitance circuit is connected to a second input terminal of the differential amplifier, and the fourth negative capacitance circuit exhibits a negative capacitance effect;
the signal acquisition circuit is **characterized in that** it further comprises a third negative capacitance circuit (C30) electrically connected between the first lead and the second lead, wherein the third negative capacitance circuit exhibits a negative capacitance effect.

10. The signal acquisition circuit according to claim 9, wherein the fourth negative capacitance circuit includes a first unit (C402) and a second unit (C404), the first unit includes a first amplifier (G2) and a third negative feedback capacitor (C46), and the second unit includes a second amplifier (G3) and a fourth negative feedback capacitor (C48), wherein the third negative feedback capacitor is connected between an input terminal of the first amplifier and an output terminal of the first amplifier, and the fourth negative feedback capacitor is connected between an input terminal of the second amplifier and an output terminal of the second amplifier.

11. The signal acquisition circuit according to claim 10, wherein a relative error between an absolute value of an equivalent capacitance value of the first unit of the fourth negative capacitance circuit and a value of a parasitic capacitance value to ground of the first lead is less than 50%, and
a relative error between an absolute value of an equivalent capacitance value of the second unit of the fourth negative capacitance circuit and a value of a parasitic capacitance to ground of the second lead is less than 50%.

12. The signal acquisition circuit according to claim 10, wherein the first amplifier and the second amplifier are amplifiers with an equal and fixed gain.

13. The signal acquisition circuit according to claim 9, wherein an common mode input impedance of the differential amplifier is greater than one hundred megaohms.

14. The signal acquisition circuit according to claim 9, wherein the differential amplifier is powered by a single power supply.

15. The signal acquisition circuit according to claim 9, wherein the fourth negative capacitance circuit includes a double-ended differential amplifier, a first negative feedback capacitor, and a second negative feedback capacitor, the first negative feedback capacitor is connected between a first input terminal of the double-ended differential amplifier and a first output terminal of the double-ended differential amplifier, and the second negative feedback capacitor is connected between a second input terminal of the double-ended differential amplifier and a second output terminal of the double-ended differential amplifier.

16. The signal acquisition circuit according to claim 15, wherein the double-ended differential amplifier is a fixed-gain amplifier.

17. A wearable device, comprising the signal acquisition circuit according to any one of the claims 1 to 16.

**Patentansprüche**

1. Signalerfassungsschaltung, umfassend:

    einen Differenzverstärker (130, 230, 530),
    eine erste Elektrode (110, 210) und eine zweite Elektrode (120, 220), wobei die erste Elektrode über eine erste Leitung (L1, L11) mit einem ersten Eingangsanschluss (A, A1) des Differenzverstärkers verbunden ist und die zweite Elektrode über eine zweite Leitung (L2, L22) mit einem zweiten Eingangsanschluss (B, B1) des Differenzverstärkers verbunden ist;
    eine erste negative Kapazitätsschaltung (C10) und eine zweite negative Kapazitätsschaltung (C20), wobei die erste negative Kapazitätsschaltung elektrisch zwischen der ersten Leitung und Erde verbunden ist und die zweite negative Kapazitätsschaltung elektrisch zwischen der zweiten Leitung und Erde verbunden ist und sowohl die erste negative Kapazitätsschaltung als auch die zweite negative Kapazitätsschaltung einen negativen Kapazitätseffekt aufweisen;
    wobei die Signalerfassungsschaltung **dadurch gekennzeichnet ist, dass** sie weiter eine dritte negative Kapazitätsschaltung (C30) umfasst, die elektrisch zwischen der ersten Leitung und der zweiten Leitung verbunden ist, wobei die dritte negative Kapazitätsschaltung einen negativen Kapazitätseffekt aufweist.

2. Signalerfassungsschaltung nach Anspruch 1, wobei ein relativer Fehler zwischen einem Absolutwert eines äquivalenten Kapazitätswerts der ersten negativen Kapazitätsschaltung und einem Wert einer parasitären Kapazität zur Erde der ersten Leitung kleiner als 50 % ist, und
    ein relativer Fehler zwischen einem Absolutwert eines äquivalenten Kapazitätswerts der zweiten negativen Kapazitätsschaltung und einem Wert einer parasitären Kapazität zur Erde der zweiten Leitung kleiner als 50 % ist.

3. Signalerfassungsschaltung nach Anspruch 1, wobei der erste Eingangsanschluss des Differenzverstärkers eine erste äquivalente Eingangskapazität aufweist und der zweite Eingangsanschluss des Differenzverstärkers eine zweite äquivalente Eingangskapazität aufweist,

    ein relativer Fehler zwischen einem Absolutwert eines äquivalenten Kapazitätswerts der ersten negativen Kapazitätsschaltung und einer Summe eines Werts einer parasitären Kapazität zur Erde der ersten Leitung und eines Werts der ersten äquivalenten Eingangskapazität kleiner als 50 % ist, und
    ein relativer Fehler zwischen einem Absolutwert eines äquivalenten Kapazitätswerts der zweiten negativen Kapazitätsschaltung und einer Summe eines Werts einer parasitären Kapazität zur Erde der zweiten Leitung und eines Werts der zweiten äquivalenten Eingangskapazität kleiner als 50 % ist.

4. Signalerfassungsschaltung nach Anspruch 1, wobei ein relativer Fehler zwischen einem Absolutwert einer äquivalenten Kapazität der dritten negativen Kapazitätsschaltung und einem Wert eines parasitären Kapazitätswerts zwischen der ersten Leitung und der zweiten Leitung kleiner als 50 % ist.

5. Signalerfassungsschaltung nach Anspruch 1, wobei die erste negative Kapazitätsschaltung einen ersten Operationsverstärker (410), einen ersten Widerstand (R1), einen zweiten Widerstand (R2) und einen ersten Kondensator (C401) einschließt und die zweite negative Kapazitätsschaltung einen zweiten Operationsverstärker, einen dritten Widerstand, einen vierten Widerstand und einen zweiten Kondensator einschließt;

    ein Umkehreingangsanschluss des ersten Operationsverstärkers über den ersten Widerstand mit der Erde verbunden ist, der Umkehreingangsanschluss des ersten Operationsverstärkers über den zweiten Widerstand mit einem Ausgangsanschluss des ersten Operationsverstärkers verbunden ist und ein Vorwärtseingangsanschluss des ersten Operationsverstärkers über den ersten Kondensator mit dem Ausgangsanschluss des ersten Operationsverstärkers verbunden ist; und
    ein Umkehreingangsanschluss des zweiten Operationsverstärkers über den dritten Widerstand mit der Erde verbunden ist, der Umkehreingangsanschluss des zweiten Operationsverstärkers über den vierten Widerstand mit einem Ausgangsanschluss des zweiten Operationsverstärkers verbunden ist und ein Vorwärtseingangsanschluss des zweiten Operationsverstärkers über den zweiten Kondensator mit dem Ausgangsanschluss des zweiten Operationsverstärkers verbunden ist.

6. Signalerfassungsschaltung nach Anspruch 5, wobei der Vorwärtseingangsanschluss des ersten Operationsverstärkers mit der ersten Leitung verbunden ist und der Vorwärtseingangsanschluss des zweiten Operationsverstärkers mit der zweiten Leitung verbunden ist.

7. Signalerfassungsschaltung nach Anspruch 1, wobei eine Gleichtakt-Eingangsimpedanz des Differenzverstärkers größer als einhundert Megaohm ist.

8. Signalerfassungsschaltung nach Anspruch 1, wobei der Differenzverstärker durch eine einzige Stromversorgung mit Strom versorgt wird.

9. Signalerfassungsschaltung, umfassend:

einen Differenzverstärker,
eine erste Elektrode und eine zweite Elektrode, und
eine vierte negative Kapazitätsschaltung (C40), wobei die erste Elektrode über eine erste Leitung mit einem ersten Eingangsanschluss (P) der vierten negativen Kapazitätsschaltung verbunden ist, die zweite Elektrode über eine zweite Leitung mit einem zweiten Eingangsanschluss (Q) der vierten negativen Kapazitätsschaltung verbunden ist, ein erster Ausgangsanschluss (M) der vierten negativen Kapazitätsschaltung mit dem ersten Eingangsanschluss des Differenzverstärkers verbunden ist und ein zweiter Ausgangsanschluss (N) der vierten negativen Kapazitätsschaltung mit einem zweiten Eingangsanschluss des Differenzverstärkers verbunden ist und die vierte negative Kapazitätsschaltung einen negativen Kapazitätseffekt aufweist;
wobei die Signalerfassungsschaltung **dadurch gekennzeichnet ist, dass** sie weiter eine dritte negative Kapazitätsschaltung (C30) umfasst, die elektrisch zwischen der ersten Leitung und der zweiten Leitung verbunden ist, wobei die dritte negative Kapazitätsschaltung einen negativen Kapazitätseffekt aufweist.

10. Signalerfassungsschaltung nach Anspruch 9, wobei die vierte negative Kapazitätsschaltung eine erste Einheit (C402) und eine zweite Einheit (C404) einschließt, die erste Einheit einen ersten Verstärker (G2) und einen dritten negativen Rückkopplungskondensator (C46) einschließt und die zweite Einheit einen zweiten Verstärker (G3) und einen vierten negativen Rückkopplungskondensator (C48) einschließt, wobei der dritte negative Rückkopplungskondensator zwischen einem Eingangsanschluss des ersten Verstärkers und einem Ausgangsanschluss des ersten Verstärkers verbunden ist und der vierte negative Rückkopplungskondensator zwischen einem Eingangsanschluss des zweiten Verstärkers und einem Ausgangsanschluss des zweiten Verstärkers verbunden ist.

11. Signalerfassungsschaltung nach Anspruch 10, wobei ein relativer Fehler zwischen einem Absolutwert eines äquivalenten Kapazitätswerts der ersten Einheit der vierten negativen Kapazitätsschaltung und einem Wert eines parasitären Kapazitätswerts zur Erde der ersten Leitung kleiner als 50 % ist, und ein relativer Fehler zwischen einem Absolutwert eines äquivalenten Kapazitätswerts der zweiten Einheit der vierten negativen Kapazitätsschaltung und einem Wert einer parasitären Kapazität zur Erde der zweiten Leitung kleiner als 50 % ist.

12. Signalerfassungsschaltung nach Anspruch 10, wobei der erste Verstärker und der zweite Verstärker Verstärker mit gleicher und fester Verstärkung sind.

13. Signalerfassungsschaltung nach Anspruch 9, wobei eine Gleichtakt-Eingangsimpedanz des Differenzverstärkers größer als einhundert Megaohm ist.

14. Signalerfassungsschaltung nach Anspruch 9, wobei der Differenzverstärker durch eine einzige Stromversorgung mit Strom versorgt wird.

15. Signalerfassungsschaltung nach Anspruch 9, wobei die vierte negative Kapazitätsschaltung einen doppelseitigen Differenzverstärker, einen ersten negativen Rückkopplungskondensator und einen zweiten negativen Rückkopplungskondensator einschließt, der erste negative Rückkopplungskondensator zwischen einem ersten Eingangsanschluss des doppelseitigen Differenzverstärkers und einem ersten Ausgangsanschluss des doppelseitigen Differenzverstärkers verbunden ist und der zweite negative Rückkopplungskondensator zwischen einem zweiten Eingangsanschluss des doppelseitigen Differenzverstärkers und einem zweiten Ausgangsanschluss des doppelseitigen Differenzverstärkers verbunden ist.

16. Signalerfassungsschaltung nach Anspruch 15, wobei der doppelseitige Differenzverstärker ein Verstärker mit fester Verstärkung ist.

17. Tragbare Vorrichtung, die die Signalerfassungsschaltung nach einem der Ansprüche 1 bis 16 umfasst.

**Revendications**

1. Circuit d'acquisition de signal comprenant :

   un amplificateur différentiel (130, 230, 530),
   une première électrode (110, 210) et une seconde électrode (120, 220), dans lequel la première électrode est connectée à une première borne d'entrée (A, A1) de l'amplificateur différentiel par l'intermédiaire d'un premier conducteur (L1, L11), et la seconde électrode est connectée à une seconde borne d'entrée (B, B1) de l'amplificateur différentiel par l'intermédiaire d'un second conducteur (L2, L22) ;
   un premier circuit de capacité négative (C10) et un deuxième circuit de capacité négative (C20), dans lequel le premier circuit de capacité négative est connecté électriquement entre le premier conducteur et la masse, et le deuxième circuit de capacité négative est connecté électriquement entre le second conducteur et la terre, et le premier circuit de capacité négative et le deuxième circuit de capacité négative présentent tous deux un effet de capacité négative ;
   le circuit d'acquisition de signal est **caractérisé en ce qu'**il comprend en outre un troisième circuit de capacité négative (C30) connecté électriquement entre le premier conducteur et le second conducteur, le troisième circuit de capacité négative présentant un effet de capacité négative.

2. Circuit d'acquisition de signal selon la revendication 1, dans lequel une erreur relative entre une valeur absolue d'une valeur de capacité équivalente du premier circuit de capacité négative et une valeur d'une capacité parasite à la masse du premier conducteur est inférieure à 50 %, et
   une erreur relative entre une valeur absolue d'une valeur de capacité équivalente du deuxième circuit de capacité négative et une valeur d'une capacité parasite à la masse du second conducteur est inférieure à 50 %.

3. Circuit d'acquisition de signal selon la revendication 1, dans lequel la première borne d'entrée de l'amplificateur différentiel présente une première capacité d'entrée équivalente, et la seconde borne d'entrée de l'amplificateur différentiel présente une seconde capacité d'entrée équivalente,

   une erreur relative entre une valeur absolue d'une valeur de capacité équivalente du premier circuit de capacité négative et une somme d'une valeur d'une capacité parasite à la masse du premier conducteur et une valeur de la première capacité d'entrée équivalente est inférieure à 50 %, et
   une erreur relative entre une valeur absolue d'une valeur de capacité équivalente du deuxième circuit de capacité négative et une somme d'une valeur d'une capacité parasite à la masse du second conducteur et une valeur de la seconde capacité d'entrée équivalente est inférieure à 50 %.

4. Circuit d'acquisition de signal selon la revendication 1, dans lequel une erreur relative entre une valeur absolue d'une capacité équivalente du troisième circuit de capacité négative et une valeur d'une valeur de capacité parasite entre le premier conducteur et le second conducteur est inférieure à 50 %.

5. Circuit d'acquisition de signal selon la revendication 1, dans lequel le premier circuit de capacité négative comprend un premier amplificateur opérationnel (410), une première résistance (R1), une deuxième résistance (R2) et un premier condensateur (C401), et le deuxième circuit de capacité négative comprend un second amplificateur opérationnel, une troisième résistance, une quatrième résistance et un second condensateur;

   une borne d'entrée inverse du premier amplificateur opérationnel est connectée à la terre par le biais de la première résistance, la borne d'entrée inverse du premier amplificateur opérationnel est connectée à une borne de sortie du premier amplificateur opérationnel par le biais de la deuxième résistance, et une borne d'entrée directe du premier amplificateur opérationnel est connectée à la borne de sortie du premier amplificateur opérationnel par le biais du premier condensateur ; et

une borne d'entrée inverse du second amplificateur opérationnel est connectée à la terre par le biais de la troisième résistance, la borne d'entrée inverse du second amplificateur opérationnel est connectée à une borne de sortie du second amplificateur opérationnel par le biais de la quatrième résistance, et une borne d'entrée directe du second amplificateur opérationnel est connectée à la borne de sortie du second amplificateur opérationnel par le biais du deuxième condensateur.

6. Circuit d'acquisition de signal selon la revendication 5, dans lequel la borne d'entrée directe du premier amplificateur opérationnel est connectée au premier conducteur, et la borne d'entrée directe du second amplificateur opérationnel est connectée au second conducteur.

7. Circuit d'acquisition de signal selon la revendication 1, dans lequel une impédance d'entrée en mode commun de l'amplificateur différentiel est supérieure à cent mégaohms.

8. Circuit d'acquisition de signal selon la revendication 1, dans lequel l'amplificateur différentiel est alimenté par une seule alimentation électrique.

9. Circuit d'acquisition de signal comprenant :

un amplificateur différentiel,
une première électrode et une seconde électrode, et
un quatrième circuit de capacité négative (C40), dans lequel la première électrode est connectée à une première borne d'entrée (P) du quatrième circuit de capacité négative par l'intermédiaire d'un premier conducteur, la seconde électrode est connectée à une seconde borne d'entrée (Q) du quatrième circuit de capacité négative par l'intermédiaire d'un second conducteur, une première borne de sortie (M) du quatrième circuit de capacité négative est connectée à la première borne d'entrée de l'amplificateur différentiel, et une seconde borne de sortie (N) du quatrième circuit de capacité négative est connectée à une seconde borne d'entrée de l'amplificateur différentiel, et le quatrième circuit de capacité négative présente un effet de capacité négative ;
le circuit d'acquisition de signal est **caractérisé en ce qu'**il comprend en outre un troisième circuit de capacité négative (C30) connecté électriquement entre le premier conducteur et le second conducteur, dans lequel le troisième circuit de capacité négative présentant un effet de capacité négative.

10. Circuit d'acquisition de signal selon la revendication 9, dans lequel le quatrième circuit de capacité négative comporte une première unité (C402) et une seconde unité (C404), la première unité comporte un premier amplificateur (G2) et un troisième condensateur de rétroaction négative (C46), et la seconde unité inclut un second amplificateur (G3) et un quatrième condensateur de rétroaction négative (C48), dans lequel le troisième condensateur de rétroaction négative est connecté entre une borne d'entrée du premier amplificateur et une borne de sortie du premier amplificateur, et le quatrième condensateur de rétroaction négative est connecté entre une borne d'entrée du second amplificateur et une borne de sortie du second amplificateur.

11. Circuit d'acquisition de signal selon la revendication 10, dans lequel une erreur relative entre une valeur absolue d'une valeur de capacité équivalente de la première unité du quatrième circuit de capacité négative et une valeur d'une valeur de capacité parasite à la masse du premier conducteur est inférieure à 50 %, et une erreur relative entre une valeur absolue d'une valeur de capacité équivalente de la seconde unité du quatrième circuit de capacité négative et une valeur d'une capacité parasite à la terre du second conducteur est inférieure à 50 %.

12. Circuit d'acquisition de signal selon la revendication 10, dans lequel le premier amplificateur et le second amplificateur sont des amplificateurs à gain égal et fixe.

13. Circuit d'acquisition de signal selon la revendication 9, dans lequel une impédance d'entrée en mode commun de l'amplificateur différentiel est supérieure à cent mégaohms.

14. Circuit d'acquisition de signal selon la revendication 9, dans lequel l'amplificateur différentiel est alimenté par une seule alimentation électrique.

15. Circuit d'acquisition de signal selon la revendication 9, dans lequel le quatrième circuit de capacité négative comporte un amplificateur différentiel à double extrémité, un premier condensateur de rétroaction négative et un second condensateur de rétroaction négative, le premier condensateur de rétroaction négative est connecté entre une première borne d'entrée de l'amplificateur différentiel à double extrémité et une première borne de sortie de l'amplificateur différentiel à double extrémité, et le second condensateur de rétroaction négative est connecté entre une seconde borne d'entrée de l'amplificateur différentiel à double extrémité et une seconde borne de sortie de l'amplificateur différentiel à double extrémité.

16. Circuit d'acquisition de signal selon la revendication 15, dans lequel l'amplificateur différentiel à double extrémité est un amplificateur à gain fixe.

17. Dispositif portable, comprenant le circuit d'acquisition de signal selon l'une quelconque des revendications 1 à 16.

**FIG. 1**

FIG. 2A

FIG. 2B

**FIG. 3**

EP 4 362 330 B1

**FIG. 4**

**FIG. 5**

**FIG. 6A**

**FIG. 6B**

**FIG. 7A**

FIG. 7B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 109981060 A

**Non-patent literature cited in the description**

- A chopper capacitive feedback instrumentation amplifier with input impedance boosting technique. **SAAD MOHAMED et al.** 2016 IEEE 59TH INTERNATIONAL MIDWEST SYMPOSIUM ON CIRCUITS AND SYSTEMS (MWSCAS). IEEE, 16 October 2016, 1-4
- 23.7 A 130dB CMRR Instrumentation Amplifier with Common-Mode Replication. **ZHANG SANFENG et al.** 2020 IEEE INTERNATIONAL SOLID- STATE CIRCUITS CONFERENCE - (ISSCC). IEEE, 16 February 2020, 356-358

- A Chopper Instrumentation Amplifier with Fully Symmetric Negative Capacitance Generation Feedback Loop and Online Digital Calibration for Input impedance Boosting. **ABDELFATTAH SAFAA et al.** 2019 IEEE 62ND INTERNATIONAL MIDWEST SYMPOSIUM ON CIRCUITS AND SYSTEMS (MWSCAS). IEEE, 04 August 2019, 774-777